# EUROPEAN PATENT SPECIFICATION

(11) **EP 4 271 945 B1**
(45) Date of publication and mention of the grant of the patent: **22.10.2025**
(21) Application number: 21854689.3
(22) Date of filing: 23.12.2021
(51) Int. Cl.: F24F 5/00, B03C 3/00, B03C 1/00, A61L 9/22, F24F 8/10, B01D 53/32, F24F 8/192

(54) **GEOTHERMAL AIR TREATMENT APPARATUS WITH ION PURIFICATION**
GEOTHERMISCHE LUFTBEHANDLUNGSVORRICHTUNG MIT IONENREINIGUNG
APPAREIL DE TRAITEMENT D'AIR GÉOTHERMIQUE AVEC PURIFICATION D'IONS

(30) Priority: 31.12.2020 EE 202000023
(43) Date of publication of application: 08.11.2023
(73) Proprietor: Air Installations OÜ, 11415 Talinn (EE)
(72) Inventor: JÜRIS, Heiki, 11415 Tallinn (EE)
(74) Representative: Koitel, Raivo
(86) International application number: PCT/IB2021/062255
(87) International publication number: WO 2022/144721

(56) References cited:
- EP-A1- 0 777 088
- WO-A1-2009/006908
- WO-A1-2009/050795
- WO-A1-2019/197859
- US-A- 4 323 113

## Description

### Technical field

This invention relates to an air treatment apparatus for premises (such as buildings, facilities, etc.) with high heat, chemical, dust, odour load and ventilation need, preheating (in winter) and cooling (in summer) and treating (purifying) the fresh supply air so that the natural air humidity would be retained also after air treatment.

### Prior art

In order to maintain the heat (in the heating period) or coolness (in summer) in premises, buildings are sealed and ventilation is provided by artificial air-conditioning. In buildings, air is treated by means of apparatuses (ventilation units with heat recovery, heating coils, heat pumps, air-conditioners) with high operation costs, drying the air (reduce indoor air quality) and consuming high levels of energy, contributing thereby also to global warming, among other. These technical possibilities also do not take into consideration air charges and reduce the quantity of negative air ions in indoor premises.

In addition to heating and cooling the indoor air of buildings there is also a need for improvement of general indoor climate of buildings, divided into three sub-concepts: microclimate, paraclimate and electroclimate. Electroclimate refers to the electrochemical composition of air in which the molecule of oxygen has the decisive role, the number of electrons in its electron shell changes easily, either giving away or gaining electrons. The concept 'aeroion' or air ion is used, which is an oxygen molecule that has either lost or gained an electron (a particle with a negative charge). Losing an electron turns the molecule into a positive ion and the gained electron into a negative ion. When oxygen is neutral or with positive ions, breathing it is not favourable for people, causing health and psychological disorders in people (in addition to gases, the city air also contains aerosol contamination with positive ion, such as dust particles, emissions from artificial materials, urban debris generated by industry and transportation and simply sprayed in the air, which in mixture with oxygen remove electrons from fresh air). However, when oxygen is with negative ions, it is a so-called aerovitamin (in nature especially near waterfalls, in pine forests and on seashores with breaking surfs). Oxygen ions with negative charge or with extra electrons are in various sizes. The smallest of these are biologically active, have high velocity and are useful for the human body. They join, forming larger ions and becoming slower. When functioning as air purifiers, they have a good property of attracting, like a magnet, pollution, positively charged particles and "drop" these from the air. For this reason it is necessary to observe the electroclimatic quality of indoor air in order to improve the indoor air of buildings ("What is fresh air" ("Mis on värske õhk"), A. Luhaste, V. Viljasoo, TM kodu & ehitus, spring 2005, p. 64-65).

The electron count in oxygen molecule changes through contact with environmental materials and also, for example breathing activity of the human body. As the outdoor air must pass through many metal pipes in ventilation systems upon entry into rooms, it loses its negative electrons on its way and so the needed negatively charged ions will not arrive in the rooms.

Known are preheating and precooling systems of supply air, also called calorifers or conditioners. For example, known is US patent US5131887 (published 21.07.1992), which is related to guiding outdoor air into a ventilation system build in the basement of the building. Air ionizers are also used in indoor premises, increasing the count of negative air ions in indoor premises.

These known apparatuses require extra energy for operation, also generating heat in the external environment during operation, meaning that they dry the air and change negative ions into positive, which all contribute to global warming.

Previously known is also Finnish patent application 20040429 (published 23.09.2005), which relates to an equipment (GTR or geothermal radiator) for utilization of geothermal heat to preheat, cool and purify air supplied to a room (building) (treat the air for positive air ions and enrich it with negative air ions). This invention includes two separate concrete underground pressure reduction chambers, which are mutually connected by pipes intended for the movement of air and covered with a ventilating soil layer (coarse material layer) to prevent the opposite energy (+) from escaping back to the atmosphere. A disadvantage of the device is that it has two separate massive concrete chambers (part of the foundation of the building), which must be cast on site and installed in the ground (completed in parallel with the building, entailing thus high construction costs of the device).

The closest prior art of the invention is international patent application WO2019/197859 A1 (published 17.10.2019), introducing a device (GTG or geothermal generator) located in a concrete chamber and a method for purification of air supplied to the room (building), removing positive air ions (aeroion) and enriching with negative air ions. This is a generator, which constantly forces positive and negative ions to mutually react, generating thereby extra energy. The device consists of an air intake chamber (upper chamber) and two pressure reduction chambers - the first pressure reduction chamber (central chamber) and the second pressure reduction chamber (lower chamber). A disadvantage of this invention is that it uses the indoor air for preheating or precooling the supply outdoor air without mixing these, after which the supply air is guided into the building. However, indoor air cannot be used in buildings with high chemical or odour load or need for ventilation with fresh air (such as bacteria-free rooms), because in such way, previously contaminated indoor air of rooms (building) is circulating in the building. Furthermore, the device uses artificial positively charged air energy provided by a separate cooling-heating unit running on additional energy.

### Disclosure of the invention

The object of this invention is an apparatus for the purification of fresh air supplied to rooms (buildings, facilities, etc.), removing positive air ions (aeroions) and enriching with negative air ions, ensuring improved quality of the indoor climate (also in relation to surrounding outdoor air) and lower operating costs of the apparatus. In an apparatus according to this invention, air is purified by natural ion exchange without the use of additional energy (electricity). In the structure of the invention this occurs in an underground apparatus, where positive and negative aeroions meet. In the apparatus, air is subjected to ion purification and temperature changes (heating in a cold season and cooling in a warm season respectively), while ion purification with negative ions creates an environment, which inhibits the vital functioning and spreading of pathogens and pollution contained in outdoor air. In addition, the apparatus allows preheating (in winter) and cooling (in summer) of the fresh supply air.

An air treatment apparatus according to this invention has four vertical parts: an above-ground part which is detachable air intake chamber with air inlet pipe/openings, inside the air intake chamber there is an aeration pipe for the exhaustion of air with positive ions from the air treatment apparatus into the ambient environment, which is connected to a fan at the top of the air intake chamber, and an under-ground part is which consists of three pressure reduction chambers surrounded by metal housing: an upper pressure reduction chamber that has an upper air treatment device comprising pre-polarized positively charged coarse material and that is connected on top to the aeration pipe passing through the air intake chamber; a lower pressure reduction chamber that has a lower air treatment device comprising pre-polarized negatively charged coarse material; a central pressure reduction chamber located between the upper pressure reduction chamber and lower pressure reduction chamber that has an inner air pipe and is connected to the air treatment device of the lower pressure reduction chamber. The air that has passed through the apparatus and which is purified and with negative ion charge and heated or cooled is extracted from the central pressure reduction chamber to the room/building through the heat recovery ventilation system of the room/building. The upper pressure reduction chamber and lower pressure reduction chamber are interconnected through lateral heat transfer tubes which are arranged vertically compared to the pressure reduction chambers. The central pressure reduction chamber is narrower than the upper pressure reduction chamber and lower pressure reduction chamber. The upper air treatment device has one cell and blind walls and the lower air treatment device has multiple cells, perforated upper walls and blind lower walls. In the upper part of the apparatus housing on the ground or its immediate vicinity is a cover/door which can be opened at the connection point of the air intake chamber and upper pressure reduction chamber and the air treatment devices of the pressure reduction chambers can be removed from the under-ground part via this. The housing of the apparatus is covered with a layer of neutral waterproofing and thermal insulation. The under-ground part of the air treatment apparatus is installed underground at the depth of constant underground temperature value. The minimum diameter of the air treatment apparatus together with the lateral heat transfer tubes is 1.6 m and height 2.1 m.

### List of figures

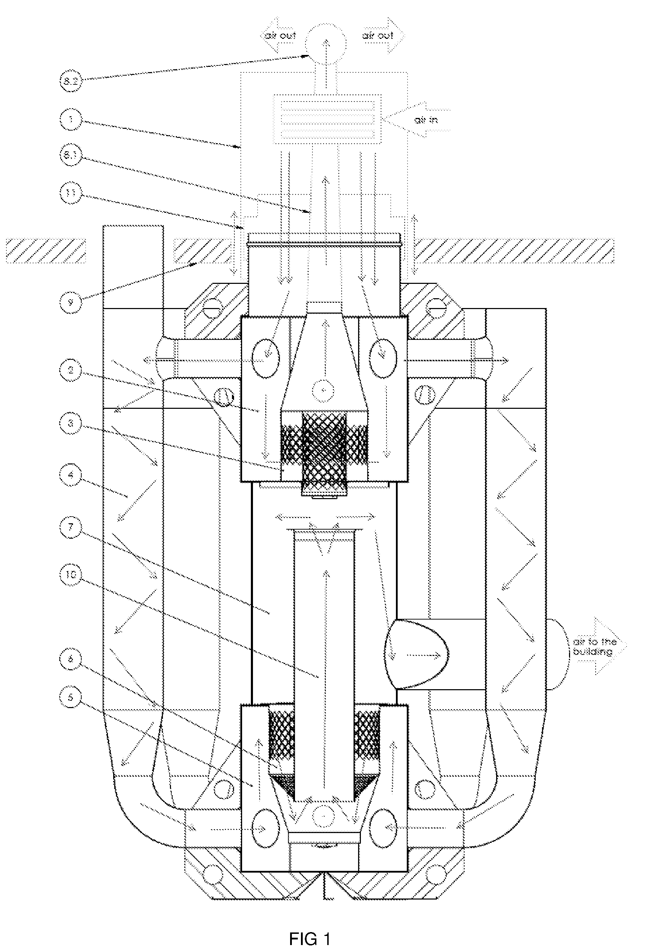

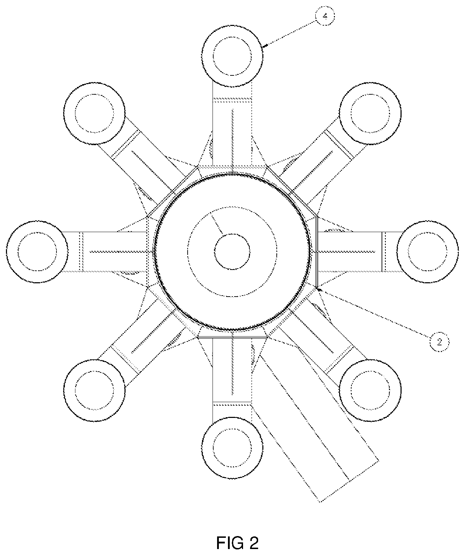

Figure fig 1 is a side view of the apparatus;
Figure fig 2 is a top view of the apparatus.

### Embodiment of the invention

This is a geothermal air treatment apparatus or geothermal radiator (GTR), which has an above-ground and underground part.

In the upper part of the apparatus is a detachable above-ground air intake chamber 1 (air pipe) for the intake of outdoor air through air inlet 1.1. In addition, the apparatus comprises three mutually vertically arranged underground pressure reduction chambers: upper pressure reduction chamber 2, lower pressure reduction chamber 5 and central pressure reduction chamber 7, the air that has entered the apparatus and is to be purified is extracted by the heat recovery ventilation system of the building (not shown in the Figure) through these and is discharged in the building. The central pressure reduction chamber 7 is between the upper pressure reduction chamber 2 and lower pressure reduction chamber 5 and is narrower than these by dimensions.

The upper pressure reduction chamber 2 and lower pressure reduction chamber 5 are interconnected through lateral heat transfer tubes 4 that have diameters and quantity calculated according to the required air volume so that the air moves inside the tubes from the upper pressure reduction chamber 2 to the lower pressure reduction chamber 5 slowly with the speed 0.5-2.5 m/s (according to law of heat conduction). Lateral heat transfer tubes 4 are installed in the ground vertically towards pressure reduction chamber 5.

Outdoor air enters through air inlet 1.1 of air intake chamber 1 into the upper pressure reduction chamber 2, where part of it is guided to the upper air treatment chamber 3 through its perforated based and part of it is guided to the lateral heat transfer tubes 4 through which the air is guided to the lower pressure reduction chamber 5. Underpressure in the upper pressure reduction chamber 2 is higher (e.g. 220 I/s) than in the lower pressure reduction chambers and there the movement of air in comparison to lower pressure reduction chambers is slower. In the upper pressure reduction chamber 2 is a single-cell upper air treatment device 3 with perforated base and blind walls, containing pre-polarized and charged with positive ions coarse material, which in the upper pressure reduction chamber 2 attracts positively charged ions contained in the outdoor air entering the apparatus (pressure reduction occurs). As a result, air which is saturated with positive ions passes through the upper air treatment device 3 and is guided through lateral heat transfer tubes 4 into the lower pressure reduction chamber 5 (e.g. in case of a balance between positive and negative ions). If positive ions are predominant, the air with unnecessary positively charged ions and unnecessary temperature that has passed through the upper air treatment device 3 is exhausted from the apparatus into the outdoor environment through aeration pipe 8.1. At the top, the upper air treatment device 3 is connected to aeration pipe 8.1 that passes through the air intake chamber 1, which again has fan 8.2 at the top end for extraction of air with unnecessary temperature and (positively) charged ions into the surrounding outdoor environment (1/3 of designed air need of the whole room/building).

In the lower pressure reduction chamber 5 is a multi-cell lower air treatment device 6 with perforated interim level and perforated upper walls and blind lower walls, where the upper cell contains pre-polarized and charged with negative ions coarse material that ensures ionization of the negatively charged outdoor air entering from the upper pressure reduction chamber 2 through lateral heat transfer tubes 4.

The lower air treatment device 6 is connected through inner air pipe 10 with the central pressure reduction chamber 7 through which air ionized with negative ions moves (e.g. at 160/200 I/s in a preferred embodiment) into the central pressure reduction chamber 7, which is either post-heated (winter) or cooled (summer) by the soil surrounding the apparatus and from where the air purified of positive ions is discharged (e.g. at 160/200 I/s in a preferred embodiment) from the apparatus into the room/building through a heat recovery ventilation system (not shown in the Figures).

The interaction between positive and negative ions generates additional energy in the central pressure reduction chamber 7, resulting in either heating (in winter) or cooling (in summer) of the air discharged into the room/building according to the supply air temperature. The lower pressure reduction chamber 5 and the central pressure reduction chamber 7 are exposed to negative ions and thus the heating or cooling effect of these chambers is higher.

In the upper part of the apparatus housing on the ground or its immediate vicinity is a cover/door 11 (preferable diameter 500-600 mm), which can be opened at the connection point of the air intake chamber and through which it is possible to remove the contents of the apparatus from the housing, allowing maintenance or replacement of the contents of the apparatus without removing/breaking the underground housing of the apparatus.

The housing of the apparatus is installed (underground) at a depth, where the ground (+) temperature remains at a constant value (8 °C) both in winter and summer (at approx. depth 2 m). In such way the ground surrounding the apparatus can either post-heat (winter) or cool (summer) the air guided from the apparatus into the room/building. On the ground, the apparatus housing is covered with a layer of neutral waterproofing and thermal insulation 9 to ensure temperature interruption between the outdoor air and the apparatus (for example, EPS 50+50 mm).

Experiments have shown that the greater the difference between outdoor temperature and ground temperature, the higher the efficiency of the apparatus according to this invention. For example: in winter with outdoor temperatures from -15 to -20 °C, the air discharged from the apparatus into the heat recovery ventilation system of the room/building is heated to 2 to 3 °C, in summer with outdoor temperatures from 25 to 30 °C, the air discharged from the apparatus into the heat recovery ventilation system is cooled to 18 to 21 °C.

The minimum diameter of an apparatus according to the preferred embodiment of the invention, including lateral heat transfer pipes is 1.6 m and height 2.1 m, which ensures ventilation in a building of up to 200 m². In a larger building, several apparatuses can be installed adjacently, according to need.

The apparatus of this invention is produced from metal, with the metal area larger than in the closest prior art solution, which again means that it produces more heat in winter and more cooling in summer. This ensures higher efficiency of the apparatus. The apparatus according to this invention can also be produced in series in workshops (for stock), it has low installation costs and it is easy to installed it to existing completed buildings.

An air treatment apparatus according to this invention is primarily suitable for production buildings with high heat, odour and dust load. For example: greenhouses, server rooms, chemistry and industrial buildings. The apparatus is also suitable for apartment buildings.

## Claims

1. Geothermal air treatment apparatus with ion purification for purification of room's/building's air which has an above-ground air intake chamber (1) with air inlet pipe/openings (1.1) and an aeration pipe (8.1); pressure reduction chambers with different dimensions arranged in the middle and lower part of the air treatment apparatus and which are connected with pipes; air treatment means; a cover (11) between the air intake chamber and one pressure reduction chamber; and the air treatment apparatus is covered with layer of waterproofing and thermal insulation, **characterized in that** the air treatment apparatus has four vertical parts: above-ground part and under-ground part, which consist of three pressure chambers with housing which are an upper pressure reduction chamber (2), a lower pressure reduction chamber (5) and a central pressure reduction chamber (7) between those two, wherein:
- the upper pressure reduction chamber (2) comprises the upper air treatment device (3) which is from top connected to an aeration pipe (8.1) that passes through the air intake chamber (1) and the aeration pipe (8.1) has a fan (8.2) in top of it, and the upper air treatment device (3) comprises coarse material which is pre-polarized and charged with positive ions;
- the lower pressure reduction chamber (5) comprises the lower air treatment device (6) which is connected with the central pressure reduction chamber (7) by means of an inner air pipe (10) and the lower air treatment device (6) comprises coarse material which is pre-polarized and charged with negative ions;
- the upper pressure reduction chamber (2) and the lower pressure reduction chamber (5) are interconnected through lateral heat transfer tubes (4);
- heated or cooled air with negative ions is directed into the room/building from the central pressure reduction chamber (7) with interaction with upper pressure reduction chamber (2) and lower pressure reduction chamber (5);
- air with non-necessary temperature and positive ions is exhausted into the ambient environment via the aeration pipe (8.1);
- the cover (11) is between the air intake chamber (1) and the upper pressure reduction chamber (2) and the air treatment devices of the pressure reduction chambers can be removed from the under-ground part via this.

2. Apparatus according to claim 1, **characterized in that** the central pressure reduction chamber (7) is narrower than the upper pressure reduction chamber (2) and the lower pressure reduction chamber (5).

3. Apparatus according to claim 1, **characterized in that** the above-ground air intake chamber (1) is detachable.

4. Apparatus according to claim 1, **characterized in that** the lateral heat transfer tubes (4) are arranged vertically in relation to the lower pressure reduction chamber (5).

5. Apparatus according to claim 1, **characterized in that** the upper air treatment device (3) has one cell and blind walls.

6. Apparatus according to claim 1, **characterized in that** the lower air treatment device (6) has multiple cells, perforated upper walls and blind lower walls.

7. Apparatus according to claim 1, **characterized in that** the minimum diameter of the air treatment apparatus together with the lateral heat transfer tubes (4) is 1.6 m and height 2.1 m.

8. Apparatus according to claim 1, **characterized in that** the under-ground part of the air treatment apparatus is installed underground at the depth of constant underground temperature value.

## Patentansprüche

1. Eine geothermische Luftbehandlungsvorrichtung mit Ionenreinigung zur Reinigung von Raum-/Gebäudeluft, die Folgendes aufweist: eine oberirdische Lufteinlasskammer (1) mit Luftzuleitungsrohr/-öffnungen (1.1) und einem Belüftungsrohr (8.1); Druckreduzierungskammern mit unterschiedlichen Abmessungen, die in dem mittleren und dem unteren Teil der Luftbehandlungsvorrichtung angeordnet sind und die mit Rohren verbunden sind; Luftbehandlungsmittel; eine Abdeckung (11) zwischen der Lufteinlasskammer und einer Druckreduzierungskammer; und die Luftbehandlungsvorrichtung ist mit einer Schicht aus Wasserabdichtung und Wärmeisolierung abgedeckt, **dadurch gekennzeichnet, dass** die Luftbehandlungsvorrichtung vier vertikale Teile aufweist: einen oberirdischen Teil und einen unterirdischen Teil, die aus drei Druckkammern mit Gehäuse bestehen, die eine obere Druckreduzierungskammer (2), eine untere Druckreduzierungskammer (5) und eine zentrale Druckreduzierungskammer (7) zwischen diesen zwei sind, wobei:
- die obere Druckreduzierungskammer (2) die obere Luftbehandlungseinrichtung (3) beinhaltet, die von oben mit einem Belüftungsrohr (8.1) verbunden ist, das durch die Lufteinlasskammer (1) verläuft, und das Belüftungsrohr (8.1) ein Gebläse (8.2) oben darauf aufweist, und die obere Luftbehandlungseinrichtung (3) grobes Material beinhaltet, das vorpolarisiert und mit positiven Ionen geladen ist;
- die untere Druckreduzierungskammer (5) die untere Luftbehandlungseinrichtung (6) beinhaltet, die mit der zentralen Druckreduzierungskammer (7) mittels eines inneren Luftrohrs (10) verbunden ist, und die untere Luftbehandlungseinrichtung (6) grobes Material beinhaltet, das vorpolarisiert und mit negativen Ionen geladen ist;
- die obere Druckreduzierungskammer (2) und die untere Druckreduzierungskammer (5) durch seitliche Wärmeübertragungsröhren (4) miteinander verbunden sind;
- erwärmte oder gekühlte Luft mit negativen Ionen in den Raum/das Gebäude von der zentralen Druckreduzierungskammer (7) mit Wechselwirkung mit der oberen Druckreduzierungskammer (2) und der unteren Druckreduzierungskammer (5) geleitet wird;
- Luft mit unbrauchbarer Temperatur und positiven Ionen über das Belüftungsrohr (8.1) in die Umgebung abgelassen wird;
- die Abdeckung (11) zwischen der Lufteinlasskammer (1) und der oberen Druckreduzierungskammer (2) ist und die Luftbehandlungseinrichtungen der Druckreduzierungskammern über diese von dem unterirdischen Teil entfernt werden können.

2. Vorrichtung gemäß Anspruch 1, **dadurch gekennzeichnet, dass** die zentrale Druckreduzierungskammer (7) schmaler als die obere Druckreduzierungskammer (2) und die untere Druckreduzierungskammer (5) ist.

3. Vorrichtung gemäß Anspruch 1, **dadurch gekennzeichnet, dass** die oberirdische Lufteinlasskammer (1) abnehmbar ist.

4. Vorrichtung gemäß Anspruch 1, **dadurch gekennzeichnet, dass** die seitlichen Wärmeübertragungsröhren (4) vertikal in Bezug auf die untere Druckreduzierungskammer (5) angeordnet sind.

5. Vorrichtung gemäß Anspruch 1, **dadurch gekennzeichnet, dass** die obere Luftbehandlungseinrichtung (3) eine Zelle und blinde Wände aufweist.

6. Vorrichtung gemäß Anspruch 1, **dadurch gekennzeichnet, dass** die untere Luftbehandlungseinrichtung (6) mehrere Zellen, perforierte obere Wände und blinde untere Wände aufweist.

7. Vorrichtung gemäß Anspruch 1, **dadurch gekennzeichnet, dass** der minimale Durchmesser der Luftbehandlungsvorrichtung zusammen mit den seitlichen Wärmeübertragungsröhren (4) 1,6 m beträgt und die Höhe 2,1 m beträgt.

8. Vorrichtung gemäß Anspruch 1, **dadurch gekennzeichnet, dass** der unterirdische Teil der Luftbehandlungsvorrichtung unterirdisch in der Tiefe eines konstanten unterirdischen Temperaturwerts installiert ist.

## Revendications

1. Appareil de traitement d'air géothermique avec purification d'ions pour la purification de l'air d'une pièce/d'un bâtiment, lequel a une chambre d'admission d'air en surface (1) avec un tuyau/des ouvertures d'entrée d'air (1.1) et un tuyau d'aération (8.1) ; des chambres de réduction de pression de différentes dimensions agencées dans la partie du milieu et inférieure de l'appareil de traitement d'air et qui sont raccordées à l'aide de tuyaux ; des moyens de traitement d'air ; un couvercle (11) entre la chambre d'admission d'air et une chambre de réduction de pression ; et l'appareil de traitement d'air est recouvert d'une couche d'imperméabilisation et d'isolation thermique, **caractérisé en ce que** l'appareil de traitement d'air a quatre parties verticales : une partie en surface et une partie dans le sous-sol, laquelle consiste en trois chambres de pression avec logement, à savoir une chambre de réduction de pression supérieure (2), une chambre de réduction de pression inférieure (5) et une chambre de réduction de pression centrale (7) entre ces deux chambres, dans lequel :
- la chambre de réduction de pression supérieure (2) comprend le dispositif de traitement d'air supérieur (3) qui est raccordé par le haut à un tuyau d'aération (8.1) qui passe à travers la chambre d'admission d'air (1) et le tuyau d'aération (8.1) a un ventilateur (8.2) au-dessus de lui, et le dispositif de traitement d'air supérieur (3) comprend un matériau grossier qui est pré-polarisé et chargé d'ions positifs ;
- la chambre de réduction de pression inférieure (5) comprend le dispositif de traitement d'air inférieur (6) qui est raccordé à la chambre de réduction de pression centrale (7) au moyen d'un tuyau d'air interne (10) et le dispositif de traitement d'air inférieur (6) comprend un matériau grossier qui est pré-polarisé et chargé d'ions négatifs ;
- la chambre de réduction de pression supérieure (2) et la chambre de réduction de pression inférieure (5) sont raccordées entre elles par l'intermédiaire de tubes de transfert de chaleur latéraux (4) ;
- l'air chauffé ou refroidi à ions négatifs est dirigé dans la pièce/le bâtiment à partir de la chambre de réduction de pression centrale (7) avec interaction avec la chambre de réduction de pression supérieure (2) et la chambre de réduction de pression inférieure (5) ;
- l'air à une température inutile et à ions positifs inutiles est évacué dans l'environnement ambiant par le biais du tuyau d'aération (8.1) ;
- le couvercle (11) se trouve entre la chambre d'admission d'air (1) et la chambre de réduction de pression supérieure (2) et les dispositifs de traitement d'air des chambres de réduction de pression peuvent être retirés de la partie dans le sous-sol par le biais de celui-ci.

2. Appareil selon la revendication 1, **caractérisé en ce que** la chambre de réduction de pression centrale (7) est plus étroite que la chambre de réduction de pression supérieure (2) et que la chambre de réduction de pression inférieure (5).

3. Appareil selon la revendication 1, **caractérisé en ce que** la chambre d'admission d'air en surface (1) est détachable.

4. Appareil selon la revendication 1, **caractérisé en ce que** les tubes de transfert de chaleur latéraux (4) sont agencés verticalement par rapport à la chambre de réduction de pression inférieure (5).

5. Appareil selon la revendication 1, **caractérisé en ce que** le dispositif de traitement d'air supérieur (3) a une cellule et des parois borgnes.

6. Appareil selon la revendication 1, **caractérisé en ce que** le dispositif de traitement d'air inférieur (6) a de multiples cellules, des parois supérieures percées et des parois inférieures borgnes.

7. Appareil selon la revendication 1, **caractérisé en ce que** le diamètre minimal de l'appareil de traitement d'air conjointement avec les tubes de transfert de chaleur latéraux (4) est de 1,6 m et la hauteur est de 2,1 m.

8. Appareil selon la revendication 1, **caractérisé en ce que** la partie dans le sous-sol de l'appareil de traitement d'air est installée dans le sous-sol à la profondeur d'une valeur de température de sous-sol constante.
